# EUROPEAN PATENT APPLICATION

(11) **EP 4 825 373 A1**
(43) Date of publication of application: **21.10.2026**
(21) Application number: 24902268.2
(22) Date of filing: 23.09.2024
(51) Int. Cl.: A61K 9/19, A61K 47/26, A61K 47/18, A61K 39/25, A61P 31/22

(54) **VACCINE LYOPROTECTANT WITHOUT ANIMAL-DERIVED PROTEIN COMPONENT AND USE THEREOF**

(30) Priority: 15.12.2023 CN 202311737409
(71) Applicant: Changchun Bcht Biotechnology Co., Jilin 130012 (CN)
(72) Inventor: LU, Jingcai, Changchun, Jilin 130012 (CN); YIN, Liguo, Changchun, Jilin 130012 (CN); YU, Yang, Changchun, Jilin 130012 (CN); ZHAO, Yonghong, Changchun, Jilin 130012 (CN); LIU, Dawei, Changchun, Jilin 130012 (CN); WEI, Wei, Changchun, Jilin 130012 (CN)
(74) Representative: Gill Jennings & Every LLP
(86) International application number: PCT/CN2024/120325
(87) International publication number: WO 2025/123842

(57) **Abstract**

The present invention relates to a vaccine lyoprotectant without animal-derived protein components, which contains the following components: 0.2 wt.%-8 wt.% of sucrose; 1.0 wt.%-10 wt.% of trehalose; 0.2 wt.%-4.0 wt.% of sodium glutamate; 0.05 wt.%-1.0 wt.% of urea; 0.05 wt.%-1.0 wt.% of arginine; 0.05 wt.%-2.0 wt.% of glucose, with the balance being a phosphate buffer solution having a pH in the range of 6.8-7.8. The present invention further relates to a semi-finished vaccine product and a lyophilized preparation prepared from the lyoprotectant.

## Description

### Technical Field

The present invention relates to the field of biomedicine, and in particular to a vaccine lyoprotectant formulation free of an animal-derived protein component, and use thereof in lyophilized live attenuated varicella vaccine formulations.

### Background Art

Varicella-zoster virus (VZV), a member of the subfamily *Alphaherpesvirinae* of the family *Herpesviridae*, is the pathogen that causes the two diseases, varicella (chickenpox) and herpes zoster (HZ). VZV is highly contagious, with a global infection rate of more than 90%. It is primarily transmitted through inhalation of infectious airborne droplets from patients with varicella or herpes zoster, or through contact. The typical symptom of varicella is a pruritic vesicular rash accompanied by mild fever; the disease exhibits distinct seasonality, occurring more frequently in winter and spring; it is characterized by high morbidity but low mortality, with a favorable prognosis. After recovery from varicella, the causative virus VZV remains latent in the ganglia; when the body's immune function declines and the VZV-specific cell-mediated immunological reaction (CMIR) weakens, the virus VZV is reactivated to cause herpes zoster.

The most effective method for preventing varicella is vaccination. Currently, varicella vaccines widely used internationally are live attenuated varicella vaccine, which have been used for many years and have been adequately proved to be safe and effective. The attenuated varicella-zoster virus strain used in the production of live attenuated varicella vaccines is primarily the attenuated Oka strain, which is recognized by the World Health Organization (WHO) and widely used. The attenuated Oka strain was isolated and attenuated by Dr. Takahashi in Japan in 1974. This attenuated strain was isolated from the varicella vesicular fluid of a 3-year-old boy named Oka as a strain of varicella-zoster virus, and was designated as the Oka strain of varicella-zoster virus. In the laboratory of Dr. Takahashi, this Oka strain was serially passaged 11 times in human embryonic lung fibroblasts (HELF) at 34°C, and then 12 times in guinea pig embryo fibroblasts (GPEF) at 37°C to further attenuate its virulence; the resulting virus was serially passaged twice in WI-38 human diploid cells to prepare the master seed virus, and finally, the master seed virus was serially passaged three times in MRC-5 (human embryonic lung fibroblast) human diploid cells to prepare the working seed virus, which represents the currently widely used attenuated Oka strain.

The process of manufacturing a live attenuated varicella vaccine primarily involves inoculating the attenuated varicella-zoster virus into a cell substrate and allowing the virus to proliferate therein; then harvesting the virus-infected cells, disrupting the cells to release the virus, and adding a freeze-drying stabilizer before lyophilization to obtain the final vaccine.

To ensure vaccine efficacy, all live attenuated varicella vaccines on the market are lyophilized formulations, which often contain animal-derived protein components such as gelatin or human serum albumin as protectants. Gelatin, as a non-human-derived component, can not only easily cause allergic reactions but also pose a potential safety risk associated with animal-derived viruses. Although human serum albumin is a human-derived component and is subject to lot release, it also poses a potential safety risk associated with plasma-derived viruses and is very expensive. In the prior art, the macromolecular substance dextran has been used to replace gelatin and human serum albumin in the formulation, but its use increases adverse reactions; alternatively, small molecule chemical components may be used as protectants, but they may compromise vaccine stability.

Chinese Patent Publication No. CN105582534A discloses a lyoprotectant for a live attenuated varicella vaccine, which comprises sucrose and sodium glutamate and is used in the preparation of a live attenuated varicella vaccine. Nevertheless, the live attenuated varicella vaccine formulated with this lyoprotectant has poor stability. After storage at 2-8°C for 24 months, the virus titer decreases significantly, approaching the lower limit of the standard requirement.

Thus, developing a protectant for a safe, effective and stable live attenuated varicella vaccine with minimal side effects is one of the urgent problems to be solved in this field.

### Summary of the Invention

In view of the above, the object of the present invention is to provide a vaccine lyoprotectant formulation free of animal-derived proteins and macromolecular substances such as dextran, its use in lyophilized live attenuated varicella vaccine formulations, and a semi-finished or finished vaccine product formulated with the same and preparation methods thereof.

The first aspect of the present invention relates to a vaccine lyoprotectant free of animal-derived protein components, which comprises the following components:
sucrose: 0.2 wt.%-8 wt.%;
trehalose: 1.0 wt.%-10 wt.%;
sodium glutamate: 0.2 wt.%-4.0 wt.%;
urea: 0.05 wt.%-1.0 wt.%;
arginine: 0.05 wt.%-1.0 wt.%;
glucose: 0.05 wt.%-2.0 wt.%; and
the balance being a phosphate buffer solution having a pH in the range of 6.8-7.8.

Preferably, the vaccine is a live attenuated varicella vaccine.

In a preferred embodiment, the protectant does not comprise macromolecular chemical components with a molecular weight greater than 500, preferably does not comprise macromolecular chemical components with a molecular weight greater than 400, and more preferably does not comprise macromolecular chemical components with a molecular weight greater than 350; examples of such macromolecular chemical components include dextran and the like.

In a preferred embodiment, the protectant comprises the following components:
sucrose: 0.4 wt.%-7 wt.%, preferably 0.5 wt.%-6 wt.%;
trehalose: 1.5 wt.%-9 wt.%, preferably 2 wt.%-8 wt.%;
sodium glutamate: 0.4 wt.%-3 wt.%, preferably 0.5 wt.%-2 wt.%;
urea: 0.08 wt.%-0.75 wt.%, preferably 0.1 wt.%-0.5 wt.%;
arginine: 0.08 wt.%-0.75 wt.%, preferably 0.1 wt.%-0.5 wt.%;
glucose: 0.08 wt.%-1.5 wt.%, preferably 0.1 wt.%-1.0 wt.%; and
the balance being a phosphate buffer solution having a pH in the range of 7.1-7.4.

In a preferred embodiment, the protectant is composed of the following components:
sucrose: 0.5 wt.%-6 wt.%;
trehalose: 2 wt.%-8 wt.%;
sodium glutamate: 0.5 wt.%-2 wt.%;
urea: 0.1 wt.%-0.5 wt.%;
arginine: 0.1 wt.%-0.5 wt.%;
glucose: 0.1 wt.%-1.0 wt.%; and
the balance being a phosphate buffer solution with having a pH in the range of 7.2-7.3.

A second aspect of the present invention relates to a method for preparing the vaccine lyoprotectant according to the first aspect of the present invention. The method comprises the following steps: weighing each component according to its content, fully dissolving the components in an appropriate amount of phosphate buffer solution, bringing the resulting solution to volume, adjusting the pH, and sterilizing by using an autoclave or a filter to give the vaccine lyoprotectant. Alternatively, the method comprises sequentially weighing each component according to its content, fully dissolving each component separately in an appropriate amount of phosphate buffer solution, mixing the resulting solutions, bringing the mixture to volume, adjusting the pH, and sterilizing by using an autoclave or a filter to give the vaccine lyoprotectant. Preferably, the vaccine is a live attenuated varicella vaccine.

A third aspect of the present invention relates to use of the vaccine lyoprotectant according to the first aspect of the present invention in the manufacture of a vaccine. Preferably, the vaccine is a live attenuated varicella vaccine.

A fourth aspect of the present invention relates to a semi-finished vaccine product, comprising a qualified vaccine bulk and the vaccine lyoprotectant according to the first aspect of the present invention. Preferably, the vaccine is a live attenuated varicella vaccine.

A fifth aspect of the present invention relates to a lyophilized vaccine formulation, comprising a qualified vaccine bulk and the vaccine lyoprotectant according to the first aspect of the present invention. Preferably, the vaccine is a live attenuated varicella vaccine. The lyophilized vaccine formulation is produced by mixing the qualified vaccine bulk with the vaccine lyoprotectant according to the first aspect of the present invention, followed by lyophilization.

A sixth aspect of the present invention relates to a method for preparing the lyophilized vaccine formulation according to the fifth aspect of the present invention, comprising the following steps: diluting a qualified vaccine bulk with the vaccine lyoprotectant according to the first aspect of the present invention based on virus titer, followed by dispensing and lyophilizing to obtain the final product. Preferably, the vaccine is a live attenuated varicella vaccine; further preferably, the vaccine has a virus titer of ≥2000 PFU/ml, such as 3000 PFU/ml or 4000 PFU/ml.

The present invention has the following beneficial effects:
1. The vaccine lyoprotectant of the present invention is free of animal-derived (including human-derived) proteins, such as gelatin and human serum albumin, and is free of macromolecular substances such as dextran; its components are all small molecular substances. Since the lyoprotectant of the present invention is free of macromolecular allergenic components, the vaccine formulated therewith is safer and less likely to cause allergic reactions. This lyoprotectant represents a new generation of lyoprotectant suitable for use in the lyophilization of live attenuated varicella vaccines.
2. The live attenuated varicella vaccine formulated with the vaccine lyoprotectant of the present invention has good stability and low cost.

### Detailed Description of the Invention

The present invention is further illustrated below with reference to specific examples so that those skilled in the art can better understand the advantages and technical effects of the present invention. Nevertheless, it should be understood that these examples are provided merely for the purpose of more detailed and specific illustration and should not be construed as limiting the present invention in any way.

In the present invention, unless otherwise specified, all operations are performed at room temperature and atmospheric pressure. The materials and operation method used in the present invention are well known in the art. The instruments and equipment used in the following examples are all conventional devices in the art; the culture media used are all commercially available conventional culture media, whose components and contents are well known to those skilled in the art.

### Example 1: Preparation of a live attenuated varicella vaccine bulk and a lyophilized vaccine formulation

1) The process for producing the live attenuated varicella vaccine primarily involves inoculating an attenuated varicella-zoster virus into a cell substrate and allowing the virus to proliferate therein; then harvesting the virus-infected cells, disrupting the cells to release the virus, adding a lyoprotectant, and then lyophilizing to yield the final vaccine. The specific procedure is as follows:

### Preparation of a live attenuated varicella vaccine bulk:

(1) The attenuated Oka strain of varicella-zoster virus was used as the seed virus, and the MRC-5 human embryonic lung diploid cells were used as the culture substrate.
(2) The MRC-5 human embryonic lung diploid cells were serially passaged in MRC-5 cell culture medium at a culture temperature of 37±1°C, and expanded to no more than passage 41; the MRC-5 cell culture medium was MEM growth medium having a pH of 7.0-7.8 and containing 5-25% calf serum;
(3) Virus infection: After the MRC-5 human embryonic lung diploid cells had grown to a monolayer, the supernatant was removed, the Oka strain of varicella-zoster virus was directly added at an MOI of 0.001 to 0.1 to infect the MRC-5 human embryonic lung diploid cells, and adsorption was allowed to proceed for ≥1 hour at 36±1°C; then maintenance medium (MEM + 2-10% bovine serum) was added, and the culture was incubated at 36°C.
(4) Harvest was initiated when the cytopathic effect reached ≥50% 3-5 days post-infection. The MRC-5 cell culture medium was discarded, and the surfaces of the MRC-5 human embryonic lung diploid cells were washed with Earle's solution with a volume more than twice that of the MRC-5 cell culture medium to remove bovine serum. The MRC-5 human embryonic lung diploid cells were digested with 0.03-0.05% EDTA solution and centrifuged at 2000 rpm for 8-10 minutes at 2-8°C, the supernatant was discarded, and the pellet was retained. The pellet was resuspended by addition of a vaccine lyoprotectant, and the varicella virus-containing cells were collected, sampled for sterility testing, and stored at -70°C.
(6) The varicella virus-containing cell suspensions that had passed the sterility testing from step (5) were combined. The resultant suspension was subjected to ultrasonic disruption at an ultrasonic frequency of 20-30 KHZ for 1-3 minutes at 2-8°C, sampled for bulk testing, and then stored at -70°C.
(7) Testing was conducted in accordance with the monograph for live attenuated varicella vaccine in the Pharmacopoeia of the People's Republic of China (2020 Edition, Volume III), with all results meeting the requirements.

Preparation of a lyophilized live attenuated varicella vaccine formulation: After passing the testing, the qualified live attenuated varicella vaccine bulk was diluted with the vaccine lyoprotectant of the present invention and mixed uniformly to achieve a virus titer of 80,000 PFU/ml, yielding a semi-finished vaccine product. The semi-finished vaccine product was immediately aliquoted at a specification of 0.5 mL per vial, and then the aliquoted semi-finished product of live attenuated varicella vaccine was subjected to a lyophilization process to obtain the lyophilized live attenuated varicella vaccine formulation. The lyophilization was performed by low-temperature vacuum freeze-drying method. After aliquoting and loading the semi-finished product into a freeze-drying chamber, the chamber was pre-frozen to -45°C, and the product temperature was lowered to below -45°C and maintained for at least 2 hours. Vacuum was applied to initiate the first stage of drying phase, i.e., sublimation drying, with vacuum control set to 20 Pa ± 5 Pa. The heat transfer oil temperature was initially set to -20°C and maintained for 8 hours. Then, the second stage of drying, i.e., desorption drying, was initiated, and the initial temperature of the heat transfer oil was set to 35°C and maintained for 6 hours before the lyophilization was complete, yielding the lyophilized live attenuated varicella vaccine formulation.

### Example 2: Preparation of three different formulations of animal-derived protein-free lyoprotectants

All components were weighed according to their respective contents (percentage by weight) in Table 1 and mixed, with the balance being a 0.01 mol/L phosphate buffer solution having a pH of 7.2, to prepare three formulations of lyoprotectants. Meanwhile, a control Lyoprotectant formulation was in-house prepared based on the formulation (0.01% sodium glutamate and 5% sucrose) in Chinese Patent Application Publication No. CN105582534A (for details, see Comparative Example 2 below).

**Table 1: Lyoprotectant formulations**

| Lyoprotectant formulations | Sucrose | Trehalose | Sodium glutamate | Urea | Arginine | Glucose |
|---|---|---|---|---|---|---|
| Formulation A | 0.5% | 8.0% | 0.5% | 0.1% | 0.5% | 0.1% |
| Formulation B | 2.0% | 5.0% | 1.0% | 0.2% | 0.3% | 0.3% |
| Formulation C | 6.0% | 2.0% | 2.0% | 0.5% | 0.1% | 1.0% |
| Control Formulation | 5% | / | 0.01% | / | / | / |

### Example 3: Protective effects of animal-derived protein-free lyoprotectants on live attenuated varicella vaccines (accelerated stability)

The three protectant formulations in Example 2 and the control protectant formulation were prepared, and the lyophilized live attenuated varicella vaccine formulations were prepared according to the process described in Example 1. The resulting vaccines were subjected to accelerated stability studies at 37°C.

**Table 2: Accelerated stability of the live attenuated varicella vaccines (37°C)**

| Test items | Standard requirements | Formulations | Test Period | | | | |
|---|---|---|---|---|---|---|---|
| | | | Day 0 | Day 7 | Day 14 | Day 21 | Day 28 |
| Appearance | It should be white loose cake; after reconstitution, it should be a clear liquid free of foreign matter | Formulation A | Pass | Pass | Pass | Pass | Pass |
| | | Formulation B | Pass | Pass | Pass | Pass | Pass |
| | | Formulation C | Pass | Pass | Pass | Pass | Pass |
| | | Control Formulation | Pass | Pass | Pass | Pass | Pass |
| Moisture content (%) | It should be not more than 3.0% | Formulation A | 1.8 | 1.8 | 2.0 | 2.1 | 2.1 |
| | | Formulation B | 2.0 | 1.8 | 2.0 | 2.3 | 2.0 |
| | | Formulation C | 1.6 | 1.6 | 1.7 | 1.8 | 1.7 |
| | | Control Formulation | 1.5 | 1.6 | 1.7 | 1.7 | 1.7 |
| Virus titer (lg PFU/dose) | It should be not less than 3.3 lg PFU/dose | Formulation A | 4.1 | 4.0 | 3.8 | 3.6 | 3.5 |
| | | Formulation B | 4.2 | 3.9 | 3.7 | 3.7 | 3.6 |
| | | Formulation C | 4.0 | 3.9 | 3.7 | 3.7 | 3.5 |
| | | Control Formulation | 4.1 | 3.6 | 3.4 | 3.3 | 3.2 |
| pH value | 6.9-7.6 | Formulation A | 7.1 | 7.1 | 7.1 | 7.2 | 7.1 |
| | | Formulation B | 7.1 | 7.1 | 7.2 | 7.2 | 7.1 |
| | | Formulation C | 7.2 | 7.3 | 7.2 | 7.3 | 7.2 |
| | | Control Formulation | 7.2 | 7.2 | 7.3 | 7.3 | 7.2 |

The above accelerated stability results show that the lyophilized live attenuated varicella vaccines formulated with the three lyoprotectant formulations exhibited good accelerated stability, with all test indicators meeting the requirements at different time points. The lyophilized live attenuated varicella vaccines formulated with the three lyoprotectant formulations showed essentially the same declining trend in virus titer: at Day 7 of the accelerated test, the titer decrease was not significant; and although the titers had declined slightly by Day 28 of the accelerated test, they all met the standard requirements. The control formulation exhibited a significant decline in titer, with the titer falling below the standard requirement (3.3 lg PFU/dose) by Day 28 of the accelerated test.

### Example 4: Protective effects of animal-derived protein-free lyoprotectants on live attenuated varicella vaccines (long-term stability)

The three protectant formulations in Example 2 and the control protectant formulation were prepared, and the lyophilized live attenuated varicella vaccine formulations were prepared according to the process described in Example 1. The resulting vaccines were subjected to long-term stability studies at 2-8°C.

**Table 3: Long-term stability of the live attenuated varicella vaccines (2-8°C)**

| Test items | Standard requirements | Formulations | Test period | | | | | |
|---|---|---|---|---|---|---|---|---|
| | | | Month 0 | Month 3 | Month 6 | Month 9 | Month 12 | Month 24 |
| Appearance | It should be white loose cake; after reconstitution, it should be a clear liquid free of foreign matter | Formulation A | Pass | Pass | Pass | Pass | Pass | Pass |
| | | Formulation B | Pass | Pass | Pass | Pass | Pass | Pass |
| | | Formulation C | Pass | Pass | Pass | Pass | Pass | Pass |
| | | Control Formulation | Pass | Pass | Pass | Pass | Pass | Pass |
| Moisture content (%) | It should be not more than 3.0% | Formulation A | 1.8 | 1.9 | 1.8 | 2.0 | 2.1 | 2.3 |
| | | Formulation B | 2.0 | 2.0 | 1.9 | 2.4 | 2.1 | 2.4 |
| | | Formulation C | 1.6 | 1.5 | 1.7 | 1.6 | 1.7 | 1.9 |
| | | Control Formulation | 1.5 | 1.6 | 1.6 | 1.7 | 1.6 | 1.8 |
| Virus titer (lg PFU/dose) | It should be not less than 3.3 lg PFU/dose | Formulation A | 4.1 | 4.1 | 4.0 | 4.0 | 4.0 | 3.9 |
| | | Formulation B | 4.2 | 4.1 | 4.0 | 4.2 | 4.1 | 4.0 |
| | | Formulation C | 4.0 | 4.2 | 4.1 | 4.1 | 4.2 | 4.0 |
| | | Control Formulation | 4.1 | 3.9 | 3.7 | 3.6 | 3.5 | 3.3 |
| pH value | 6.9-7.6 | Formulation A | 7.1 | 7.1 | 7.2 | 7.1 | 7.1 | 7.2 |
| | | Formulation B | 7.1 | 7.2 | 7.2 | 7.1 | 7.2 | 7.2 |
| | | Formulation C | 7.2 | 7.3 | 7.3 | 7.2 | 7.3 | 7.2 |
| | | Control Formulation | 7.2 | 7.3 | 7.3 | 7.3 | 7.2 | 7.3 |
| Sterility Test | No microbial growth should be observed. | Formulation A | Pass | NA | NA | NA | Pass | Pass |
| | | Formulation B | Pass | NA | NA | NA | Pass | Pass |
| | | Formulation C | Pass | NA | NA | NA | Pass | Pass |
| | | Control Formulation | Pass | NA | NA | NA | Pass | Pass |

| | | | | | | | | |
|---|---|---|---|---|---|---|---|---|
| Note: NA means not applicable | | | | | | | | |

The above long-term stability results show that the lyophilized live attenuated varicella vaccines formulated with the three lyoprotectant formulations exhibited good long-term stability during the 24 months, with all test indicators meeting the requirements at different time points. The lyophilized live attenuated varicella vaccines formulated with the three lyoprotectant formulations showed no significant decrease in virus titers at Month 24, and all met the quality requirements. The control formulation exhibited a significant decline in titer, with the titer falling below the standard requirement (3.3 lg PFU/dose) by Month 24.

### Comparative Example 1

Chinese Patent Application Publication No. CN105582534A discloses a lyoprotectant for a live attenuated varicella vaccine, which comprises sucrose and sodium glutamate and is used in the preparation of a live attenuated varicella vaccine. In this Example, the vaccine lyoprotectant free of gelatin, human serum albumin and dextran as described in CN105582534A was used as comparative lyoprotectant II (as described in Examples 2-4) to prepare a lyophilized live attenuated varicella vaccine (prepared according to the relatively preferred formulation B of said patent application). The specific steps are as follows:
Comparative lyoprotectant II: 0.01% sodium glutamate and 5% sucrose, with the balance being a 0.01 mol/L phosphate buffer solution having a pH of 7.2. It was prepared using the same method as described in Example 2 of the present application.

The same batch of live attenuated varicella vaccine bulk prepared according to the method for preparing a live attenuated varicella vaccine bulk as described in Example 1 of the present application was taken and tested. After passing the testing, the qualified bulk was diluted with the comparative lyoprotectant II and the three vaccine lyoprotectant formulations of the present invention, respectively, and mixed uniformly to achieve a virus titer of 80,000 PFU/ml, yielding semi-finished vaccine products. The semi-finished vaccine products were immediately aliquoted at a specification of 0.5 mL per vial, and then the aliquoted semi-finished products of live attenuated varicella vaccine were subjected to a lyophilization process to obtain lyophilized live attenuated varicella vaccine formulations. The lyophilization was performed by low-temperature vacuum freeze-drying method. After aliquoting and loading the semi-finished products into a freeze-drying chamber, the chamber was pre-frozen to -45°C, and the product temperature was lowered to below -45°C and maintained for at least 2 hours. Vacuum was applied to initiate the first stage of drying, i.e., sublimation drying, with vacuum control set to 20 Pa ± 5 Pa. The heat transfer oil temperature was initially set to -20°C and maintained for 8 hours. Then, the second stage of drying, i.e., desorption drying, was imitated, and the initial temperature of the heat transfer oil was set to 35°C and maintained for 6 hours before the lyophilization was complete, yielding lyophilized live attenuated varicella vaccine formulations.

A comparison of the resulting lyophilized live attenuated varicella vaccine formulations indicates that the live attenuated varicella vaccine formulated with the comparative lyoprotectant II has poor stability; after storage at 37°C for 28 days, the virus titer showed a remarkable decline, falling below the standard requirement (3.3 lg PFU/dose); after storage at 2-8°C for 24 months, the virus titer decreased significantly, approaching the lower limit of the standard requirement (3.3 lg PFU/dose). Nevertheless, the live attenuated varicella vaccines formulated with the vaccine lyoprotectants of the present invention, which are free of dextran, gelatin, and human serum albumin, have a well-defined composition and good stability; after storage for 24 months, the virus titer did not decrease significantly, and all other indicators also met the requirements.

In view of the foregoing, the lyoprotectant formulations of the present invention, which are free of animal-derived protein components, are suitable for live attenuated varicella vaccines. The lyophilized live attenuated varicella vaccine formulations prepared therewith exhibit good stability and are free of macromolecular allergenic components, thereby reducing adverse reactions and exhibiting a good safety profile.

The scope of protection of the present invention is defined by the claims. The Examples are merely illustrative of the concepts and principles of the present invention, and it should be understood that these specifically described embodiments do not constitute limitations on the scope of the present invention.

## Claims

1. A vaccine lyoprotectant free of animal-derived protein components, comprising the following components:
sucrose: 0.2 wt.%-8 wt.%;
trehalose: 1.0 wt.%-10 wt.%;
sodium glutamate: 0.2 wt.%-4.0 wt.%;
urea: 0.05 wt.%-1.0 wt.%;
arginine: 0.05 wt.%-1.0 wt.%;
glucose: 0.05 wt.%-2.0 wt.%; and
the balance being a phosphate buffer solution having a pH in the range of 6.8-7.8.

2. The vaccine lyoprotectant according to claim 1, wherein the lyoprotectant does not comprise macromolecular chemical components with a molecular weight greater than 500, preferably does not comprise macromolecular chemical components with a molecular weight greater than 400, and more preferably does not comprise macromolecular chemical components with a molecular weight greater than 350.

3. The vaccine lyoprotectant according to claim 1 or claim 2, wherein the vaccine lyoprotectant comprises the following components:
sucrose: 0.4 wt.%-7 wt.%, preferably 0.5 wt.%-6 wt.%;
trehalose: 1.5 wt.%-9 wt.%, preferably 2 wt.%-8 wt.%;
sodium glutamate: 0.4 wt.%-3 wt.%, preferably 0.5 wt.%-2 wt.%;
urea: 0.08 wt.%-0.75 wt.%, preferably 0.1 wt.%-0.5 wt.%;
arginine: 0.08 wt.%-0.75 wt.%, preferably 0.1 wt.%-0.5 wt.%;
glucose: 0.08 wt.%-1.5 wt.%, preferably 0.1 wt.%-1.0 wt.%; and
the balance being a phosphate buffer solution having a pH in the range of 7.1-7.4.

4. The vaccine lyoprotectant according to claim 3, wherein the vaccine lyoprotectant is composed of the following components:
sucrose: 0.5 wt.%-6 wt.%;
trehalose: 2 wt.%-8 wt.%;
sodium glutamate: 0.5 wt.%-2 wt.%;
urea: 0.1 wt.%-0.5 wt.%;
arginine: 0.1 wt.%-0.5 wt.%;
glucose: 0.1 wt.%-1.0 wt.%; and
the balance being a phosphate buffer solution with having a pH in the range of 7.2-7.3.

5. A method for preparing the vaccine lyoprotectant according to any one of claims 1-4, comprising the following steps:
weighing each component according to its content,
fully dissolving the components in an appropriate amount of phosphate buffer solution,
bringing the resulting solution to volume,
adjusting the pH, and
sterilizing by using an autoclave or a filter to give the vaccine lyoprotectant; or
sequentially weighing each component according to its content,
fully dissolving each component separately in an appropriate amount of phosphate buffer solution,
mixing the resulting solutions,
bringing the mixture to volume,
adjusting the pH, and
sterilizing by using an autoclave or a filter to give the vaccine lyoprotectant.

6. Use of the vaccine lyoprotectant according to any one of claims 1-4 in the manufacture of a vaccine.

7. A semi-finished vaccine product, comprising a vaccine bulk and the vaccine lyoprotectant according to any one of claims 1-4.

8. A lyophilized vaccine formulation, comprising a vaccine bulk and the vaccine lyoprotectant according to any one of claims 1-4.

9. A method for preparing the lyophilized vaccine formulation according to claim 8, comprising the following steps:
diluting a qualified vaccine bulk with the vaccine lyoprotectant according to any one of claims 1-4 based on virus titer, followed by dispensing and lyophilizing to obtain the lyophilized vaccine formulation.

10. The vaccine lyoprotectant according to any one of claims 1-4, or the method according to claim 5, or the use according to claim 6, or the semi-finished vaccine product according to claim 7, or the lyophilized vaccine formulation according to claim 8, or the method according to claim 9, wherein the vaccine is a live attenuated varicella vaccine.
